# EUROPEAN PATENT SPECIFICATION

(11) **EP 2 242 531 B1**
(45) Date of publication and mention of the grant of the patent: **22.06.2016**
(21) Application number: 09784532.5
(22) Date of filing: 11.02.2009
(51) Int. Cl.: A61M 31/00, A61M 25/00, A61B 90/00, A61M 25/09, A61B 17/00, A61B 17/34

(54) **Neurosurgical catheter**
Neurochirurgischer Katheter
Cathéter neurochirurgicale

(30) Priority: 13.02.2008 GB 0802634
(43) Date of publication of application: 27.10.2010
(73) Proprietor: Renishaw (Ireland) Limited, Swords (IE)
(72) Inventor: DERRICK, Hugo George, Stroud, Gloucestershire GL5 3DR (GB); STRATTON, Matthew David Frederick, Stroud, Gloucestershire GL5 2EJ (GB); FIELDER, Paul David, Stroud, Gloucestershire GL6 8LF (GB)
(74) Representative: Dunn, Paul Edward
(86) International application number: PCT/GB2009/000376
(87) International publication number: WO 2009/101397

(56) References cited:
- WO-A-03/002170
- WO-A-03/077785
- US-A- 5 643 197
- US-A1- 2004 199 128
- US-A1- 2007 088 295

## Description

The present invention relates to medical catheters and in particular to neurosurgical catheters for insertion directly into the brain parenchyma of a subject.

There are many situations where there is a requirement to deliver therapeutic agents directly to specific targets within the brain parenchyma using implanted catheters. Furthermore, many of these therapeutic agents will cause unwanted side effects if delivered to healthy parts of the brain. Examples of treating abnormalities of brain function include the acute infusion of Gamma-amino-buturic-acid agonists into an epileptic focus or pathway to block transmission, and the chronic delivery of opiates or other analgesics to the peri-aqueductal grey matter or to thalamic targets for the treatment of intractable pain. Also, cytotoxic agents can be delivered into or near a brain tumour. Intraparenchymal infusion can also be used to deliver therapeutic agents to brain targets that can not be delivered systemically because they will not cross the blood-brain barrier. For example, the treatment of patients with Parkinson's disease, Alzheimer's disease, head injury, stroke and multiple sclerosis may be carried out by the infusion of neurotrophic factors (e.g. GDNF) to protect and repair failing or damaged nerve cells. Neurotrophins may also be infused to support neural grafts transplanted into damaged or malfunctioning areas of the brain in order to restore function.

A number of neurosurgical catheters have been developed previously that can be guided (e.g. using a stereoguide) to desired target sites within the brain parenchyma. For example, it has been described previously in WO2003/077785 how a fine neurosurgical catheter formed from carbothane can be inserted into the brain using a guide tube arrangement of the type described in US6609020. In one embodiment described in WO2003/077785, a guide tube is inserted into the brain along a guide wire using a stereotactic placement technique. This allows the distal end of the guide tube to be accurately located just short of the desired brain target. A fine neurosurgical catheter, reinforced by a fine tungsten guide wire, is then inserted into the implanted guide tube and passed along the guide tube until it reaches the distal end thereof. The catheter tip then exits the guide tube and catheter insertion is continued until the catheter tip reaches the desired target. The fine guide wire is then withdrawn from the catheter lumen leaving the catheter in situ.

The use of fused silica catheters for the delivery of drugs in to the brain parenchyma has also been proposed previously. Fused silica catheters are, however, relatively brittle and tend to fracture if bent. This makes such catheters unsuitable for long term implantation within a subject. It has also been proposed previously, for example see WO02/070036, to provide an intraparenchymal catheter having a reinforced porous membrane segment at its distal end for draining or delivering fluid. A catheter comprising a flexible portion and a rigid tip portion is described in WO2003/002170.

According to the present invention, there is provided a neurosurgical kit as defined in appended claim 1. Preferred embodiments are defined in the dependent claims. The kit includes a neurosurgical catheter for insertion into the brain parenchyma of a subject and a neurosurgical guide tube as described in more detail below. The catheter comprises a flexible tube and has a stiff, non-porous, tip comprising at least one fluid delivery aperture.

The present invention thus provides a neurosurgical catheter arranged for insertion into the brain parenchyma of a subject. The catheter comprises a length of flexible tubing and has a stiff tip or tip region that can be accurately located at, or adjacent to, a required target point or region within the brain. The catheter may comprise one or more lumens as required and, when implanted, may delivery any type of therapeutic agent or fluid directly to a target region within the brain. The stiff tip of the catheter is non-porous and comprises at least one fluid delivery aperture (e.g. a single aperture at the distal end of the tip) which is preferably in fluid communication with one or more lumens thereof.

A catheter having a stiff tip in accordance with the present invention has the advantage that it can be accurately guided to a target site within the brain parenchyma. In particular, the stiff tip will not be significantly deflected from the required insertion direction even when passed through virgin brain tissue or tough matter such as brain tumours or the like. A catheter of the present invention thus has the advantage of not requiring any additional reinforcement (e.g. using a stiffening wire or cannula) during implantation, although some reinforcement (e.g of the flexible tube) may still be provided if required. Providing mechanical stiffness only at the tip also means that the catheter can be handled easily during the implantation procedure; for example, the flexible tube that leads to the stiff tip can still be readily bent without snapping and can thus be easily routed (e.g. subcutaneously) from the point where the catheter exits the skull to an implanted fluid pump or the like. The present invention thus combines the guiding related advantages that have been found to be associated with forming a catheter from stiff material with the ease of implantation that is associated with using a catheter comprising flexible tubing. A catheter of the present invention can also be fully primed before insertion thereby preventing the introduction of air in to the brain.

A catheter of the present invention is particularly suited for use in combination with guide tube devices such as those described in WO2003/077785 and US6609020. As mentioned above, WO2003/077785 describes how a guide tube can be stereotactically implanted in the brain so that its distal end is just short of a desired target. A fine flexible catheter, reinforced by an even finer tungsten wire, is then inserted into the brain parenchyma through the guide tube. During catheter insertion, the catheter tip exits the distal end of the guide tube and is forced a short distance through brain tissue to the desired target. It has, however, been found that in some instances the tip of the catheter described in WO2003/077785 can still deviate from the axis of insertion defined by the longitudinal axis of the guide tube during such an implantation process. Even relatively small deviations from the identified target site are undesirable as they can significantly reduce treatment efficacy and may cause unwanted damage to sensitive regions of the brain. These deviations from the required target have been found to be a particular problem when the catheter has a small outside diameter (thereby requiring the use of a very thin tungsten wire) and/or when the tip has to be inserted into relatively tough tissue (such as a brain tumour or the like). The removal of the tungsten guide wire after catheter implantation without disturbing catheter placement can also prove problematical. The present invention, through the provision of the stiff catheter tip, overcomes the need to use a reinforcing guide wire during catheter implantation whilst also allowing accurate guiding of the catheter tip to the required target. The present invention thus avoids certain problems that can arise when using catheters of the type described in WO2003/077785.

In addition, providing stiffness only at the catheter tip overcomes the various problems that have been found to be associated with the long term implantation of known fused silica catheters that are stiff along their entire length. In particular, a catheter of the present invention may be implanted and the flexible tube bent in the vicinity of the skull bone to allow the catheter to be subcutaneously buried for long term implantation. This should be contrasted to known fused silica catheters that have been found to fracture and fail when subjected to bending for prolonged periods. The present invention thus also mitigates implantation problems that are associated with known fused silica catheters.

As outlined above, the stiff tip of the catheter is stiffer than the flexible tube of the catheter. The flexible tube can thus, conveniently, be bent through a tighter radius of curvature than the stiff tip for a given applied force or before failing. The stiff tip of the catheter may be formed in many different ways. For example, a coating or chemical treatment could be applied to the distal end of the flexible tube to form the stiff tip. Material could also be added or embedded at the distal end of the flexible tube to form the stiff tip; for example, the stiffness at the tip could be provided by co-extruded carbon fibres etc. If material is drawn to form the catheter, the variation in stiffness could also be provided by altering the draw ratio used to form the tip and the flexible tube. A stiff tip could also be provided by modifying the cross-section of a region at the distal end of the tube or mechanically structuring such a region (e.g. by providing ribs). The stiff tip may also be stiff when implanted, but reduce in stiffness after implantation. For example, a dissolvable stiffening coating may be provided or a material may be used that becomes more flexible when implanted (e.g. due to warming to body temperature).

As outlined above, the stiff tip is non-porous and comprises at least one fluid delivery aperture. A single fluid aperture may be provided at the distal end of the stiff tip and/or one or more apertures may be provided in the side of the stiff tip and/or in the side of the flexible tube. The stiff, non-porous, tip is preferably formed from a substantially impermeable material in which the at least one fluid delivery aperture is formed. The stiff tip preferably comprises one or a few discrete fluid delivery apertures. The stiff tip preferably comprises no more than 1000 fluid delivery apertures, more preferably no more than 100 fluid delivery apertures, more preferably no more than 10 fluid delivery apertures, more preferably no more than 5 fluid delivery apertures and more preferably no more than 2 fluid delivery apertures.

The stiff tip is non-porous and therefore does not include multiple minute pores through which a therapeutic substance may diffuse, but instead provides one or more fluid dispensing apertures through which fluid can be expelled under pressure. Advantageously, each fluid delivery aperture provided at the stiff tip permits a fluid to be dispensed at pressure in a defined direction. For example, the at least one fluid delivery aperture is conveniently configured for use in the convection enhanced delivery of therapeutic substances to a target site within the brain parenchyma. Preferably, the one or more fluid delivery apertures provided at the stiff tip each have a dimension greater than 50µm, more preferably greater than 0.1mm, more preferably greater than 0.2mm, more preferably greater than 0.3mm and more preferably greater than 0.5mm. The one or more fluid delivery apertures are preferably at least equal in size to the internal diameter of the lumen(s) of the stiff tip.

Advantageously, the stiff tip comprises a stiff tube. The stiff tube may conveniently be attached, directly or indirectly, to the distal end of the flexible tube. For example, the stiff tube may be glued to the flexible tube or the flexible tube and stiff tube may comprise complimentary, mating, connectors. Preferably, the proximal end of the stiff tube is retained within the lumen of the flexible tube. For example, the proximal end of the stiff tube may be inserted into the distal end of the flexible tube. The stiff tube may be partially or completely located within the lumen of the flexible tube. Advantageously, the distal end of the stiff tube protrudes from the distal end of the flexible tube. Preferably, the distal end of the flexible tube retains the inserted stiff tube by a friction grip. For example, the distal end of the flexible tube may be plastically and/or elastically deformable to retain the inserted stiff tube. In a preferred embodiment, the distal end of the flexible tube may be heated during manufacture so as to securely capture the stiff tube by a shrink fit.

The stiff tube may comprise any suitable, mechanically stiff, material. The material of the stiff tube is advantageously mechanically stiffer than the material of the flexible tube. Conveniently, the Young's modulus or modulus of elasticity of the stiff tube is greater than the Young's modulus of the flexible tube. Advantageously, the material of the stiff tube has a Young's modulus that is at least one, at least two or at least three orders of magnitude greater than the Young's modulus of the material of the flexible tube. Advantageously, the stiff tube has a Young's modulus greater than or equal to 5GPa, more preferably greater than 10GPa, more preferably greater than 20GPa, more preferably greater than 50GPa and more preferably greater than 70GP. The flexible tube preferably has a Young's modulus less than 5GPa, more preferably less than 1GPa, more preferably less than 500MPa, more preferably less than 250MPa, more preferably less than 100MPa and more preferably less than 25MPa. In the preferred embodiment described below, a stiff tube formed from fused silica (having a Young's modulus of around 73GPa) is used with a flexible Carbothane 72DB20 tube (having a Young's modulus of around 24MPa).

A stiff tube providing the stiff tip may conveniently comprise a ceramic, such as synthetic fused Silica, Zirconia, Tungsten Carbide etc. It should also be noted that the term ceramic used herein takes the well known meaning laid down by the American Society for the Testing of Materials (ASTM), namely as being a glazed or unglazed body of crystalline, or partly crystalline structure, or of glass, which body is produced from essentially inorganic, non-metallic substances and either is formed from a molten mass which solidifies on cooling, or is formed and simultaneously or subsequently matured by the action of the heat. The ASTM definition of ceramic as used herein thus includes amorphous materials such as glass and synthetic fused silica.

The stiff tube may conveniently comprise a metal, such as Titanium or Aluminium, or a combination of ceramic and metal, such as a ceramic-metal matrix (e.g. cemented Tungsten Carbide). The present invention thus provides an intraparenchymal catheter comprising a fluid delivery tube having a fluid dispensing tip portion at its distal end, the fluid dispensing tip portion being mechanically stiffer than the fluid delivery tube.

Preferably, the whole of the stiff tip is located within the volume defined by the brain parenchyma when the catheter is implanted in the brain of a subject. In other words, the stiff tip is preferably arranged to be no longer than the depth of catheter implantation within the brain. The length of the stiff tip is preferably less than 10cm, more preferably less 5cm, more preferably 3cm or less and more preferably 2cm or less. The stiff tip is preferably at least 0.5cm long, more preferably at least 1cm long and more preferably at least 2cm long. Conveniently, the stiff tip is approximately 2cm long.

Advantageously, the flexible tube comprises a polymer. For example, the flexible tube may comprise PTFE, FEP, polyurethane, polypropylene or HDPE. The distal end of the flexible tube is, when the catheter is implanted in a subject, preferably contained within the brain parenchyma of the brain of a subject. The proximal end of the flexible tube is, when the catheter is implanted in a subject, preferably located outside the brain parenchyma.

The proximal end of the flexible tube may be connected to a supply tube. The supply tube may also be flexible and may have an outside diameter that is greater than the flexible tube. The connection between the flexible tube and the supply tube is conveniently located outside of the brain parenchyma and is preferably located outside the skull. Advantageously, fixing means are provided for securing the flexible tube of the catheter in place (e.g. by fixing it to the skull) after implantation; this ensures that the catheter tip does not deviate from the desired position within the brain parenchyma. The supply tube may, for example, be connected to the flexible tube by a connector or hub that is secured (e.g. screwed) to the outside of the skull and subcutaneously buried under the scalp.

The catheter may be designed for long term implantation and is thus preferably fabricated from materials that are suitable for long term implantation. Advantageously, at least one of the flexible tube and the stiff tip are formed solely from virally inert materials; this is a particular advantage when delivering virus based therapies.

Advantageously the flexible tube is a fine flexible tube having an outer diameter of no more than 2mm, more preferably no more than 1mm, more preferably no more than 0.8mm and more preferably no more than 0.5mm. Conveniently, the stiff tip has an outer diameter of no more than 2mm, more preferably no more than 1mm, more preferably no more than 0.8mm and more preferably no more than 0.5mm. Conveniently, the outside diameter of the stiff tip is no greater than the outside diameter of the flexible tube. If the catheter comprises a stiff tube attached to the distal end of the flexible tube, the outside diameter of the stiff tube is preferably less than the outside diameter of the flexible tube. Advantageously, the outside diameter of the stiff tube is smaller than the outside diameter of the flexible tube by at least 5 percent, more preferably by at least 10 percent and more preferably by at least 25 percent.

It should also be noted that the catheter is preferably passively insertable (i.e. it is preferably not actively steerable). For example, the catheter preferably does not include any kind of steering mechanism for altering the orientation of the tip relative to flexible tube. Conveniently, the catheter provides only a fluid delivery function. The catheter may include only a single lumen, or a may comprise a plurality of lumens.

The present invention comprises a neurosurgical kit comprising; a neurosurgical catheter as described above and a neurosurgical guide tube device, wherein the neurosurgical guide tube device comprises a guide channel (e.g. formed by an elongate guide tube) through which the neurosurgical catheter can be passed. The neurosurgical guide tube device is preferably of the type described previously in US6609020 or WO2003/077785.

Advantageously, the proximal end of the guide tube device comprises a head portion for attachment to a hole formed in the skull of a subject. The catheter may then be arranged such that, when the guide tube and catheter are implanted in a subject, the flexible tube of the catheter passes through, and is bent in the vicinity of, the head portion. Advantageously, the guide channel of the guide tube device is longer than the stiff tip of the catheter. In this manner, only the flexible tube of the catheter needs to be bent thereby removing any requirement for the stiffer material forming the tip to be bent thereby preventing any stress being exerted on the stiff tip after implantation.

Conveniently, the stiff tip of the catheter, when implanted, is arranged to only partially protrude from the distal end of the guide channel of the guide tube. In other words, part of the stiff tip may remain located within the guide channel of the guide tube after implantation. Conveniently, the outer diameter of the catheter is less than the internal diameter of the guide channel and such relative diameters are preferably arranged so that the catheter fits snugly within the guide channel. The guide channel of the guide tube thus acts to guide the tip to the desired target even after the distal end of the tip has exited the guide channel. Based on the teachings contained herein, a skilled person would thus be able to select the relative lengths of the stiff tip and the guide tube for the particular surgical procedure being performed; this selection would vary from subject to subject and would take into account the required proximity of the guide tube to the desired target and the depth of the target within the brain. It should also be noted that the guide tube and/or catheter may be manufactured as standard lengths and tailored (e.g. cut by the surgeon) to the required size before or during the surgical procedure.

The kit may also include other components. For example, a subcutaneous drug delivery pump and/or additional fluid tubing may be provided. A stereoguide for implanting the guide tube device may also be provided as part of the kit.

Also disclosed is a neurosurgical catheter which comprises a fluid delivery tube having a fluid dispensing tip portion at its distal end, wherein the fluid dispensing tip portion is formed from a different, preferably non-porous, material than the fluid delivery tube. The fluid dispensing tip portion may be formed from a stiff material, such as ceramic (zirconia, fused silica etc) of the type described above. The fluid dispensing tip may, when the catheter is implanted, be completely located within the brain parenchyma. If the catheter is required for acute infusion, the fluid delivery tube may be formed from a rigid material (e.g. zirconia). Advantageously, the fluid delivery tube is formed from a flexible material (e.g. a polymer) as described above.

According to a third aspect of the disclosure, a catheter comprises a flexible tube and a stiff tip, characterised in that the stiff tip comprises a stiff tube comprising ceramic material. A ceramic of the type described above (e.g. fused silica, Tungsten Carbide etc) may be conveniently used. The stiff tip may also comprise a metal; for example, a ceramic-metal matrix such as cemented Tungsten Carbide may be used.

According to a fourth aspect of the disclosure, a method of manufacturing a catheter comprises the steps of (i) taking a flexible tube and a stiff tube, and (ii) affixing the stiff tube to the distal end of the flexible tube, wherein step (ii) comprises at least partially inserting the stiff tube into the lumen of the flexible tube or abutting the stiff tube to the distal end of the flexible tube.

Advantageously, step (ii) comprises permanently securing the stiff tube to the flexible tube. This may be performed by, for example, using a shrink fit, an adhesive etc. The stiff tube may comprise metal and/or ceramic. Preferable, the stiff tube is formed from silica. Preferable, the stiff tube is non-porous.

According to a fifth aspect of the disclosure, a method of delivering a therapeutic substance to a target with the brain parenchyma of a subject is provided. The method comprises the steps of (i) taking a catheter comprising a flexible tube and a stiff tip, and (ii) inserting the catheter into the brain parenchyma of a subject.

Advantageously, step (ii) comprises inserting the catheter into the brain parenchyma through a previously implanted guide tube device. An initial step may thus be performed of implanting a guide tube device, such as a guide tube device of the type described previously in US6609020 or WO2003/077785, in the brain parenchyma of a subject. During the implantation of the guide tube device, its distal end may be located (just) short of the required target within the brain parenchyma.

Advantageously, step (ii) comprises passing the catheter through the previously implanted guide tube device until the stiff tip of the catheter reaches the desired target within the brain parenchyma. Conveniently, the stiff tip may be guided by the guide tube device as it exits therefrom and is moved towards the target. In other words, the stiff tip may protrude sufficiently from the end of the guide tube device to reach the desired target whilst ensuring enough of the stiff tip is retained within the guide tube to provide guidance to the target. Preferable, the stiff tip is non-porous.

Once implanted, the step (iii) may be performed of delivering a therapeutic substance to the brain parenchyma via the implanted catheter. A catheter may be implanted whenever delivery of a therapeutic substance is required or it may advantageously remain implanted for the long term (e.g. for months or years).

Also described herein is a neurosurgical catheter for insertion into the brain parenchyma of a subject, wherein the catheter comprises a flexible tube and is characterised by having a stiff tip. The stiff tip may or may not be porous and may have any one or more of the additional features that are outlined above.

The invention will now be described, by way of example only, with reference to the accompanying drawings in which;
Figure 1 illustrates a prior art neurosurgical catheter and guide tube arrangement,
Figure 2 illustrates a catheter of the present invention, and
Figure 3 illustrates a catheter of the present invention inserted into an implanted guide tube.

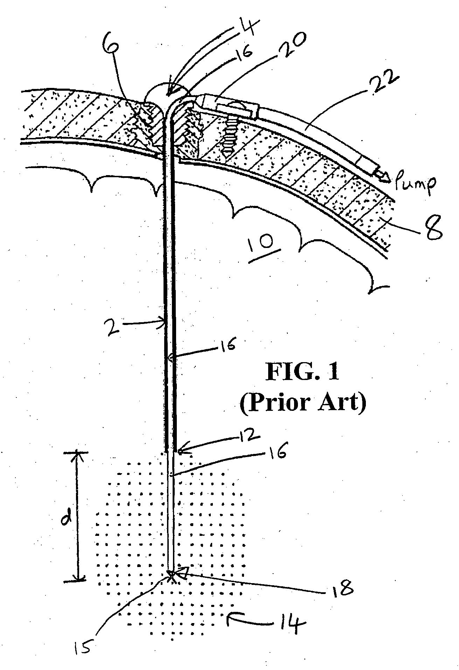

Referring to figure 1, a prior art implanted fluid delivery system of the type described in WO2003/077785 is illustrated.

The fluid delivery system comprises a guide tube device comprising an elongate guide tube 2 having a head portion 4 at its proximal end. The head portion 4 has an external thread 6 to allow attachment to a burr hole formed in the skull bone 8 of a subject. The guide tube device is inserted stereotactically into the brain parenchyma 10 using a stereoguide device. In particular, the guide tube device can be accurately inserted in the brain along a predefmed axis of insertion such that it's distal end 12 is located just short (by a distance d) of a target point 15. More details concerning accurate (e.g. stereotactic) insertion of the guide tube can be found elsewhere; for example, see WO2003/077784, WO2003/077785 and US6609020.

After the guide device has been implanted, a flexible catheter is inserted through the head portion 4 and into the guide tube 2. The flexible catheter comprises a length of fine tubing 16 having an outside diameter of 1mm or less. During implantation, the fine tubing 16 is inserted into the guide tube 2 and advanced therethrough until the distal end 18 of the fine tube 16 protrudes a distance "d" from the distal end 12 of the guide tube 2 and thereby reaches the target point 15. As described in WO2003/077785, the fine tube 16 is flexible and is typically reinforced by a guide wire (not shown) during implantation to prevent the catheter significantly deviating from the required axis of insertion as it is exits the distal end 12 of the guide tube 2 and is driven towards target point 15. Once implanted, the guide wire is withdrawn from the catheter leaving the fine tube 16 in situ.

The fine tube 16 of the catheter is connected to a hub 20 that is screwed to the outside of the skull 8. A supply tube 22 is in fluid communication with the fine tube 16 via a channel formed in the hub 20. The supply tube 22 may receive fluid from an implanted drug pump, the fluid then being routed along the fine tube 16 to the target volume 14. The catheter and guide tube device are arranged to be long term implantable thereby allowing drug delivery, either continuously or intermittently, over prolonged periods of time.

Although the prior art neurosurgical catheter system described above with reference to figure 1 typically enables accurate catheter placement, it has been found by the present inventors that it can sometimes suffer from a number of problems. For example, the use of a fine tube 16 (e.g. having an outer diameter of 1mm or less) means that only a relatively small diameter guide wire can be used to stiffen the catheter during insertion. This means that the distal end 18 of the catheter can still wander off course during implantation, especially when insertion into tough tissue (such a brain tumour or cyst) is required. It has also been found that the process of removing a fine guide wire from the fine tubing 16 can prove difficult to perform in a surgical environment and in particular that the process of guide wire removal can sometimes reduce the accuracy with which the distal end 18 is located relative to the target point 15.

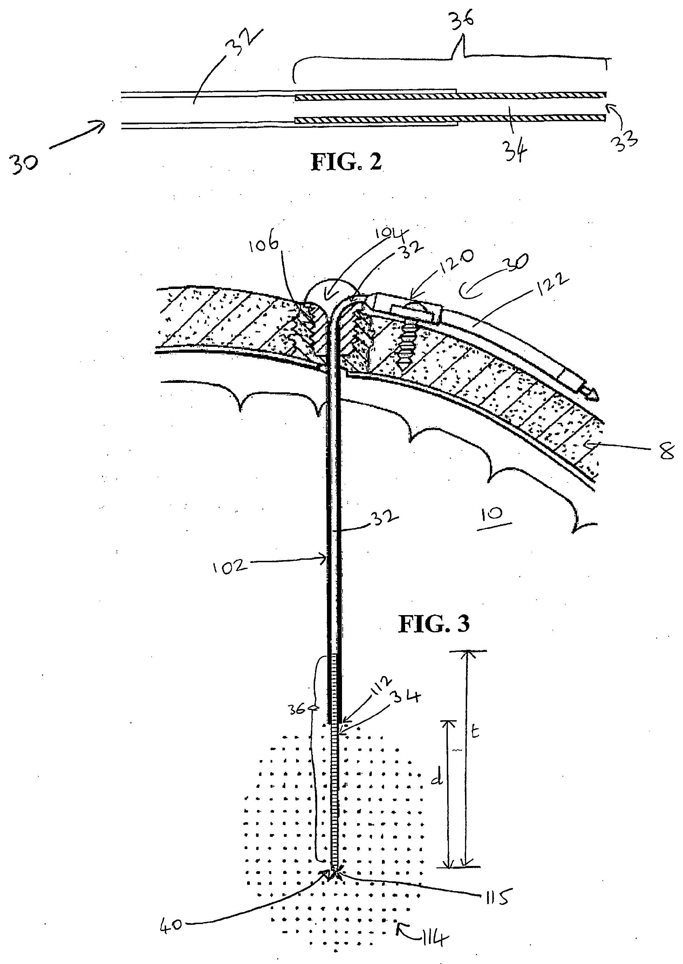

Referring to figure 2, an improved catheter 30 according to the present invention is shown. In particular, figure 2 shows a view of the tip region 36 of a neurosurgical catheter 30 of the present invention.

The catheter 30 comprises a length of flexible tube 32. The flexible tube 32 may be formed from a polymer such as carbothane 72DB20, which has a Young's modulus of elasticity of around 24MPa, and preferably has an outside diameter of around 1mm or less. The flexible tube may, however, be formed from other materials and may have an outside diameter greater than 1mm if required. A short (e.g. 20mm long) stiff tube 34 is attached to the distal end of the flexible tube 32. The stiff tube 34 may be formed from any suitably stiff material, but in the present example it comprises a length of synthetic fused silica tubing; fused silica having a Young's modulus of around 73GPa. The catheter 30 thus has a tip region or tip 36 that is mechanically stiffer than the flexible tube 32 through which fluid is supplied to the tip 36. A single fluid dispensing aperture 33, having a diameter equal to the diameter of the lumen of the stiff tube 34, is provided at the distal end of tip region 36. Although not shown, it should be noted that one or more fluid dispensing apertures may alternatively or additionally be provided in the side walls of the stiff tube 34 at the tip region 36. The proximal end of the flexible tube 32 may optionally be attached to a supply tube via a hub, although these are not shown in figure 2 for clarity.

It can thus be seen that, in this embodiment of the invention, a single lumen is provided through the catheter and that fluid will exit the catheter through a single aperture located at the distal end of the stiff tube 34. It should, however, be noted that multiple lumen variants of the catheter may be provided. Furthermore, the fluid aperture may be located in a different position to that shown in figure 2; for example, an aperture may be provided on the side of the stiff tube 34. If necessary, more than one fluid aperture may also be provided.

The catheter 30 can be fabricated using any one of a number of techniques. In a preferred embodiment, the catheter 30 is fabricated by inserting 15mm of a 20mm long fused silica stiff tube 34 into the lumen of a carbothane flexible tube 32. Heating a small (e.g. 3mm) region at the end of the flexible tube 32 causes the polymer to shrink thereby capturing the stiff tube 34 and securing the stiff tube 34 in place. To aid bonding between the stiff and flexible tubes, the stiff tube 34 may optionally comprise an outer coating of polyamide or similar material that is compatible with the fused silica of the stiff tube. Heating the flexible tube then causes the carbothane of the flexible tube to be welded to the polyamide coating thereby forming a secure grip. Furthermore, the heat shrink process provides a smooth or tapered transition (not shown in figure 3) from the flexible tube to stiff tube which ensures that the outer surface of the catheter is free from projections or protrusions thereby minimising the damage to brain tissue during catheter insertion. If necessary, an adhesive may also be applied to ensure the stiff tube 34 does not detach from the flexible tube 32.

It should be noted that a catheter of the present invention may be formed using many other materials For example, the stiff tube could be formed from a different ceramic material (e.g. Tungsten Carbide which has a Young's modulus of around 680GPa) or a metal (e.g. Aluminium which has a Young's modulus of around 70GPa). Cemented Tungsten Carbide, having a Young's modulus of around 650GPa, could also be used to provide the stiff tube. Similarly, the flexible tube could be formed from different flexible materials. For example, the Young's modulus of FEP, PEEK, Polypropylene and Carbothane all fall within the range of 4MPa (Carbothane 75A) to 4GPa (PEEK) and are thus suitable for use as the flexible tube.

A variety of different fabrication techniques could also be employed to make the catheter. For example, the stiff tube 34 may also be attached to the flexible tube 32 in a variety of different ways (e.g. using an adhesive etc). The length of stiff tube 34 that is inserted into the flexible tube 32 can also be selected as required; for example, the stiff tube 34 may be completely or partially contained with the flexible tube 32. As described in more detail below, the length of the stiff tip 36 may be tailored for the particular surgical procedure. It should also be noted that it is not essential that the stiff tip is formed by attaching a stiff tube to a flexible tube; the stiff tip may also be formed in other ways, such as by hardening (e.g. by heating or exposing to UV) the distal end of the flexible tube or modifying the cross-section at the distal end of the tube.

Referring to figure 3, implantation of a catheter of the present invention in a subject will be described.

In common with prior art arrangements of the type described with reference to figure 1, a guide tube device comprising a guide tube 102 and a head portion 104 is firstly implanted in a subject (e.g. a person or an animal) using known stereotactic techniques. The guide tube 102 thus defines an axis of insertion to a target point 115 for delivery of a therapeutic agent to a target volume 114 within the brain parenchyma 10. A thread 106 provided on the head portion 104 firmly anchors the guide device to the skull bone 8 of the subject.

The catheter 30 of the present invention is inserted into the guide tube 102 through the head portion 104. The tip 36 of the catheter (formed by the stiff tube 34) is then fed along the guide tube 102 towards the target volume 114. The catheter 30 is inserted into the guide device until the distal end 40 of the catheter tip 36 extends a distance d from the distal end of the guide tube 102. This distance d can be set by providing a mark or other indicator (e.g. a graticule or scale) on the flexible tube 32 and a corresponding mark on the head portion 104; alignment of these marks indicates that the distal end of the catheter has advanced the required distance d from the distal end of the guide tube 102. Imaging techniques may also or alternatively be used during implantation to identify catheter tip position.

The distance d is selected to be less than the length t of the stiff tip 36. Furthermore, the guide tube 102 is arranged to have an internal diameter that is only slightly larger than the outside diameter of the flexible tube 32 of the catheter. In this manner, the stiff tube 34 is guided along the axis of insertion defined by the guide tube 102 and, importantly, such guidance is still provided even when the distal end 40 of the catheter 30 exits the guide tube 102. The inherent stiffness of the catheter tip 36 thus accurately guides the tip to the target point 115 without the need to use any kind of wire or cannula to reinforce the catheter. The problems associated with using, and removing, a guide wire are thus mitigated thereby making the catheter implantation process simpler and quicker whilst providing high targetting accuracy. Furthermore, a catheter of the present invention can be fully primed before insertion thereby preventing the introduction of air in to the brain.

Once the distal end 40 of the catheter 30 has been placed at the target point 115, the flexible tube 32 can be bent through a right angle at the head portion 104 of the guide device. The flexible tube is sufficiently bendable to be routed (without fracturing) through a right angle in the vicinity of the skull bone (e.g. within the head portion 104 of the guide tube device) to allow subcutaneous burying of the catheter. It should be noted that it is the flexible tube 32 that is bent and there is no need to bend the stiff tube 34; this prevents any fracturing that could result if the whole catheter was formed from a stiff material. The present invention can thus be seen to combine the guidance advantages of using a stiff material with the ease of tube routing that is provided by flexible polymer tubes.

In the present embodiment, the proximal end of the flexible tube 32 is attached to a hub 120 that is screwed to the skull bone 8 of the patient thereby securing the catheter in place. A supply tube 122 for supplying fluid from an associated (e.g. implanted) drug pump is also connected to the flexible tube 32 via the hub 120. The supply tube 122 and hub 120 can then be subcutaneously buried under the scalp making the catheter suitable for long term implantation within a patient. It should, however, be noted that the hub 120 and supply tube 122 are not essential parts of the invention and merely provide a convenient means for routing fluid to the flexible tube 32 for onward delivery to the target volume 114. The proximal end of the flexible tube 32 may be connected, permanently or whenever required, to any (e.g. implanted or external) fluid source when fluid delivery through the catheter is required. Fluid delivery by convection enhanced delivery may be advantageously performed using the catheter. The length of the flexible tube 32 and/or tube 122 may thus be selected to permit the required fluid connections.

It should also be noted that the catheter of the present invention can also allow the distance d between the distal end 112 of the guide tube 102 and the required target point 115 to be increased if required without significantly degrading targeting accuracy. Increasing this distance can reduce the amount of damage to brain tissue and can also reduce fluid reflux along the interface between the brain tissue and the guide tube. The tip length t and/or the distance d between the distal end 112 of the guide tube 102 and the target point 115 can thus be varied as required on a patient-to-patient basis to provide the optimum treatment regimen.

It is also important to note that the catheter of the present invention can be used with a different type of guide tube than that described above and may even be used without any kind of guide tube device not according to the invention. For example, a catheter of the present invention alone may be inserted to the brain parenchyma. It should also be noted that although the above examples refer to delivery of therapeutic agents (e.g. drugs, viruses etc) through the catheter, it would also be possible to collect a fluid using the catheter.

The above described catheter is particularly suited for use in neurosurgical applications where catheter insertion directly into the brain parenchyma through a hole in the skull is required. The catheter can, however, also be used for other medical applications. For example, it may be used in applications where fluid needs to be delivered to an accurately defined target within an organ (e.g. to the liver, kidneys etc). The skilled person would thus be aware of the numerous applications for the catheter described herein.

## Claims

1. A neurosurgical kit, comprising;
a neurosurgical catheter (30) for insertion into the brain parenchyma (10) of a subject, the catheter (30) comprising a flexible tube (32) and a stiff, non-porous, tip (36) comprising at least one fluid delivery aperture,
**characterised in that** the kit also includes a neurosurgical guide tube device (102) comprising a guide channel through which the neurosurgical catheter (30) can be passed, wherein, when implanted, the stiff tip (36) of the catheter is arranged to only partially protrude from the distal end of the guide channel of the guide tube device (102).

2. A kit according to claim 1, wherein the stiff tip (36) of the catheter (30) comprises a stiff tube (34), the stiff tube being attached to the distal end of the flexible tube (32).

3. A kit according to claim 2, wherein the proximal end of the stiff tube (34) is located within the lumen of the flexible tube (32) and the distal end of the stiff tube (34) protrudes from the distal end of the flexible tube (32).

4. A kit according to claim 3, wherein the distal end of the flexible tube (32) retains the inserted stiff tube (34) by a friction grip.

5. A kit according to any one of claims 2 to 4, wherein the stiff tube (34) comprises at least one of a ceramic and a metal.

6. A kit according to claim 5, wherein the stiff tube (34) comprises fused silica.

7. A kit according to any preceding claim, wherein the stiff tip (36) of the catheter is mechanically stiffer than the flexible tube (32).

8. A kit according to any preceding claim wherein, when the catheter (30) is implanted in the brain parenchyma (10) of a subject, the whole of the stiff tip (36) is located within the volume defined by the brain parenchyma.

9. A kit according to any preceding claim, wherein the length of the stiff tip (36) is less than 10cm and more than 0.5cm.

10. A kit according to any preceding claim, wherein the flexible tube (32) comprises a polymer.

11. A kit according to any preceding claim, wherein the flexible tube (32) and the stiff tip (36) are formed solely from virally inert materials.

12. A kit according to any preceding claim, wherein the flexible tube (32) is a fine flexible tube having an outer diameter of no more than 1mm.

13. A kit according to any preceding claim, wherein the proximal end of the guide tube device (102) comprises a head portion (104) for attachment to a hole formed in the skull of a subject, wherein, when the guide tube device (102) and catheter (30) are implanted in a subject, the flexible tube (32) of the catheter passes through, and is bent in the vicinity of, the head portion (104).

14. A kit according to any preceding claim, wherein the guide channel of the guide tube device (102) is longer than the stiff tip (36) of the catheter.

## Patentansprüche

1. Neurochirurgische Ausstattung, umfassend:
einen neurochirurgischen Katheter (30) zum Einsetzen in das Gehirnparenchym (10) eines Subjektes, wobei der Katheter (30) ein flexibles Rohr (32) und eine steife, nicht poröse Spitze (36) umfasst, die zumindest eine Fluidlieferdurchbrechung umfasst,
**dadurch gekennzeichnet, dass** die Ausstattung auch eine neurochirurgische Führungsrohrvorrichtung (102) aufweist, die einen Führungskanal umfasst,
durch den der neurochirurgische Katheter (30) geführt werden kann, wobei bei Implantation die steife Spitze (36) des Katheters so angeordnet ist, dass sie nur teilweise von dem distalen Ende des Führungskanals der Führungsrohrvorrichtung (102) vorragt.

2. Ausstattung nach Anspruch 1, wobei die steife Spitze (36) des Katheters (30) ein steifes Rohr (34) umfasst, wobei das steife Rohr an dem distalen Ende des flexiblen Rohrs (32) befestigt ist.

3. Ausstattung nach Anspruch 2, wobei das proximale Ende des steifen Rohrs (34) in dem Lumen des flexiblen Rohrs (32) angeordnet ist und das distale Ende des steifen Rohres (34) von dem distalen Ende des flexiblen Rohres (32) vorragt.

4. Ausstattung nach Anspruch 3, wobei das distale Ende des flexiblen Rohres (32) das eingesetzte steife Rohr (34) durch einen Reibungsgriff hält.

5. Ausstattung nach einem der Ansprüche 2 bis 4, wobei dass steife Rohr (34) eine Keramik und/oder ein Metall umfasst.

6. Ausstattung nach Anspruch 5, wobei das steife Rohr (34) geschmolzenes Silica umfasst.

7. Ausstattung nach einem der vorhergehenden Ansprüche, wobei das steife Rohr (36) des Katheters mechanisch steifer als das flexible Rohr (62) ist.

8. Ausstattung nach einem der vorhergehenden Ansprüche, wobei, wenn der Katheter (30) in das Gehirnparenchym (10) eines Subjektes implantiert ist, die gesamte steife Spitze (36) in dem Volumen angeordnet ist, das von dem Gehirnparenchym definiert ist.

9. Ausstattung nach einem der vorhergehenden Ansprüche, wobei die Länge der steifen Spitze (36) kleiner als 10 cm und größer als 0,5 cm ist.

10. Ausstattung nach einem der vorhergehenden Ansprüche, wobei das flexible Rohr (32) ein Polymer umfasst.

11. Ausstattung der vorhergehenden Ansprüche, wobei das flexible Rohr (32) und die steife Spitze (36) ausschließlich aus viral inerten Materialen geformt sind.

12. Ausstattung nach einem der vorhergehenden Ansprüche, wobei das flexible Rohr (32) ein feines flexibles Rohr ist, das einen Außendurchmesser von nicht größer als 1 mm aufweist.

13. Ausstattung nach einem der vorhergehenden Ansprüche, wobei das proximale Ende der Führungsrohrvorrichtung (102) einen Kopfabschnitt (104) zur Befestigung an einem Loch umfasst, das in dem Schädel eines Subjekts geformt ist, wobei, wenn die Führungsrohrvorrichtung (102) und der Katheter (30) in einem Subjekt platziert sind, das flexible Rohr (32) des Katheters durch den Kopfabschnitt (104) verläuft und in der Nähe davon gebogen ist.

14. Ausstattung nach einem der vorhergehenden Ansprüche, wobei der Führungskanal der Führungsrohrvorrichtung (102) länger als die steife Spitze (36) des Katheters ist.

## Revendications

1. Kit neurochirurgical, comprenant :
un cathéter neurochirurgical (30) destiné à être inséré dans le parenchyme cérébral (10) d'un sujet, le cathéter (30) comprenant un tube flexible (32) et une pointe non poreuse rigide (36) comprenant au moins une ouverture de distribution de fluide,
**caractérisé en ce que** le kit comporte également un dispositif de tube de guidage neurochirurgical (102) comprenant un canal de guidage à travers lequel le cathéter neurochirurgical (30) peut passer, où, lorsqu'elle est implantée, la pointe rigide (36) du cathéter est agencée pour faire saillie uniquement en partie depuis l'extrémité distale du canal de guidage du dispositif de tube de guidage (102).

2. Kit selon la revendication 1, dans lequel la pointe rigide (36) du cathéter (30) comprend un tube rigide (34), le tube rigide étant fixé à l'extrémité distale du tube flexible (32).

3. Kit selon la revendication 2, dans lequel l'extrémité proximale du tube rigide (34) est située à l'intérieur de la lumière du tube flexible (32) et l'extrémité distale du tube rigide (34) fait saillie depuis l'extrémité distale du tube flexible (32).

4. Kit selon la revendication 3, dans lequel l'extrémité distale du tube flexible (32) maintient le tube rigide inséré (34) par une prise par frottement.

5. Kit selon l'une quelconque des revendications 2 à 4, dans lequel le tube rigide (34) comprend au moins l'un(e) d'une céramique et d'un métal.

6. Kit selon la revendication 5, dans lequel le tube rigide (34) comprend de la silice fondue.

7. Kit selon l'une des revendications précédentes, dans lequel la pointe rigide (36) du cathéter est mécaniquement plus rigide que le tube flexible (32).

8. Kit selon l'une des revendications précédentes, dans lequel, lorsque le cathéter (30) est implanté dans le parenchyme cérébral (10) d'un sujet, la totalité de la pointe rigide (36) est située à l'intérieur du volume défini par le parenchyme cérébral.

9. Kit selon l'une des revendications précédentes, dans lequel la longueur de la pointe rigide (36) est inférieure à 10 cm et supérieure à 0,5 cm.

10. Kit selon l'une des revendications précédentes, dans lequel le tube flexible (32) comprend un polymère.

11. Kit selon l'une des revendications précédentes, dans lequel le tube flexible (32) et la pointe rigide (36) sont formés seulement à partir de matériaux viralement inertes.

12. Kit selon l'une des revendications précédentes, dans lequel le tube flexible (32) est un tube flexible fin ayant un diamètre extérieur inférieur ou égal à 1 mm.

13. Kit selon l'une des revendications précédentes, dans lequel l'extrémité proximale du dispositif de tube de guidage (102) comprend une partie de tête (104) destinée à être fixée à un trou formé dans le crâne d'un sujet, où, lorsque le dispositif de tube de guidage (102) et le cathéter (30) sont implantés chez un sujet, le tube flexible (32) du cathéter traverse la partie de tête (104) et est plié au voisinage de celle-ci.

14. Kit selon l'une des revendications précédentes, dans lequel le canal de guidage du dispositif de tube de guidage (102) est plus long que la pointe rigide (36) du cathéter.
